# EUROPEAN PATENT APPLICATION

(11) **EP 4 400 053 A1**
(43) Date of publication of application: **17.07.2024**
(21) Application number: 22867350.5
(22) Date of filing: 06.09.2022
(51) Int. Cl.: A61B 5/377

(54) **BIOLOGICAL INFORMATION RECORDING DEVICE AND BIOLOGICAL INFORMATION RECORDING PROGRAM**

(30) Priority: 07.09.2021 JP 2021145252
(71) Applicant: Sumitomo Pharma Co., Ltd., Osaka 541-0045 (JP)
(72) Inventor: YOSHIMIZU, Minoru, Osaka-shi, Osaka 541-0045 (JP); OCHIAI, Yasushi, Tokyo 103-6012 (JP); FUKUSHIMA, Yumiko, Tokyo 103-6012 (JP); SADAMOTO, Yuiko, Tokyo 103-6012 (JP); IZUNO, Toru, Tokyo 103-0014 (JP); KOYAMA, Hiroaki, Tokyo 103-0014 (JP)
(74) Representative: Zimmermann & Partner Patentanwälte mbB
(86) International application number: PCT/JP2022/033450
(87) International publication number: WO 2023/038028

(57) **Abstract**

A biological information recording device as an example of the present disclosure includes: an acquisition processor configured to acquire biological information on a subject, which is detected by a biological information sensor; and a recording processor configured to record the biological information acquired by the acquisition processor, and to further record, when a designation operation that designates a task to be carried out by the subject is performed by an observer who observes the subject during recording of the biological information, the task in association with the biological information.

## Description

### Technical Field

The present disclosure relates to a biological information recording device, and a biological information recording program.

### Background Art

Conventionally, there have been known biological information sensors such as electroencephalographs worn on the heads of subjects to detect electroencephalograms of the subjects have been known.

### Citation List

### Patent Literature

Patent Literature 1: U.S. Patent No. 9867571

### Summary of Invention

### Technical Problem

The conventional biological information sensors as described above can be used to record biological information on subjects performing one or more tasks such as a test or assignment which may be a mental or physical load, and are imposed for, for example, checking cognitive functions or like. In this case, it is desirable to easily associate the biological information and the task and record them.

Accordingly, one of the problems to be solved by the present disclosure is to provide a biological information recording device and a biological information recording program capable of easily associating biological information and a task and recording them.

### Solution to Problem

A biological information recording device as an example of the present disclosure includes: an acquisition processor configured to acquire biological information on a subject, which is detected by a biological information sensor; and a recording processor configured to record the biological information acquired by the acquisition processor, and to further record, when a designation operation that designates a task to be carried out by the subject is performed by an observer who observes the subject during recording of the biological information, the task in association with the biological information.

Further, a biological information recording program as another example of the present disclosure causes a computer to execute: acquiring biological information on a subject, which is detected by a biological information sensor, and recording the acquired biological information, and further recording, when a designation operation that designates a task to be carried out by the subject is performed by an observer who observes the subject during recording of the biological information, the task in association with the biological information.

### Advantageous Effect of Invention

According to the biological information recording device and the biological information recording program of the present disclosure, the biological information and the task can be simply associated and recorded.

### Brief Description of Drawings

[Figure 1] Figure 1 is an exemplary and schematic view illustrating an outline of an electroencephalogram recording device and an electroencephalograph according to an embodiment.
[Figure 2] Figure 2 is an exemplary and schematic block diagram illustrating functions of the electroencephalogram recording device according to the embodiment.
[Figure 3] Figure 3 is an exemplary and schematic diagram illustrating an example of a top image according to the embodiment.
[Figure 4] Figure 4 is an exemplary and schematic diagram illustrating an example of a preparation image according to the embodiment.
[Figure 5] Figure 5 is an exemplary and schematic diagram illustrating an example of a recording image for a right-hander according to the embodiment.
[Figure 6] Figure 6 is an exemplary and schematic diagram illustrating an example of a recording image for a left-hander according to the embodiment.
[Figure 7] Figure 7 is an exemplary and schematic diagram illustrating an example of a history image according to the embodiment.
[Figure 8] Figure 8 is an exemplary and schematic diagram illustrating an example of a confirmation image according to the embodiment.
[Figure 9] Figure 9 is an exemplary and schematic diagram illustrating an example of a recording format of data according to the embodiment.
[Figure 10] Figure 10 is an exemplary and schematic diagram illustrating an example of a recording format of a task according to the embodiment.
[Figure 11] Figure 11 is an exemplary and schematic flowchart illustrating a process executed when the top image is displayed in the embodiment.
[Figure 12] Figure 12 is an exemplary and schematic flowchart illustrating a process executed when the preparation image is displayed in the embodiment.
[Figure 13] Figure 13 is an exemplary and schematic flowchart illustrating a process executed when the recording image is displayed in the embodiment.
[Figure 14] Figure 14 is an exemplary and schematic flowchart illustrating a process executed when the history image is displayed in the embodiment.
[Figure 15] Figure 15 is an exemplary and schematic flowchart illustrating a process executed when the confirmation image is displayed in the embodiment.
[Figure 16] Figure 16 is an exemplary and schematic block diagram illustrating an example of a hardware configuration of a computer that realizes the electroencephalogram recording device according to the embodiment.

### Description of Embodiment

A biological information recording device and a biological information recording program according to the present disclosure is described below based on the drawings of the embodiment. A configuration of the embodiment described below and operations and effects brought about by the configuration are merely examples, and the present disclosure is not limited to the following description contents.

Also, although ordinal numbers such as "first", and "second" are used as necessary in the present disclosure, these ordinal numbers are used for convenience of identification and do not indicate a particular priority.

### <Embodiment>

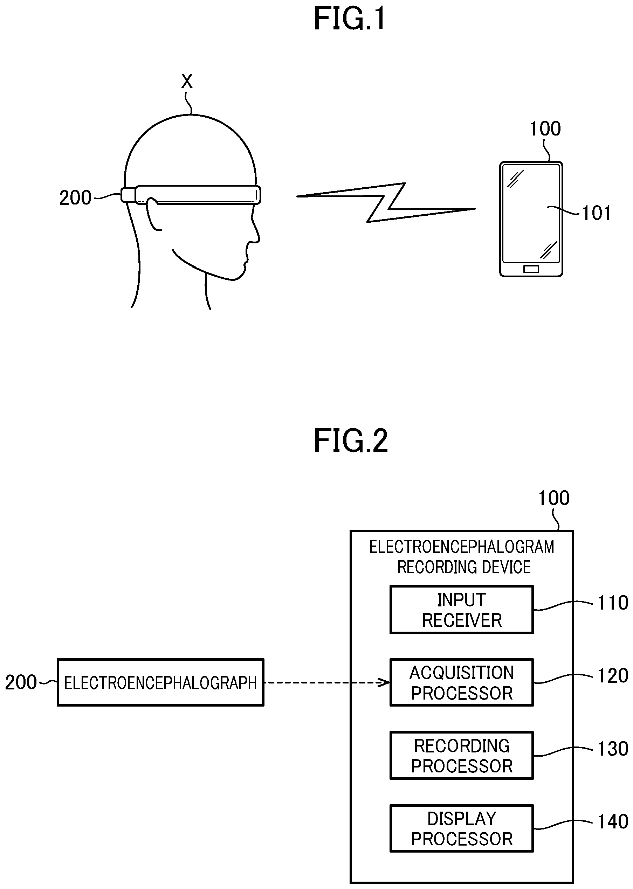

Figure 1 is an exemplary and schematic view illustrating an outline of an electroencephalogram recording device 100 and an electroencephalograph 200 according to the embodiment. The electroencephalogram recording device 100 is an example of a "biological information recording device" of the present disclosure, and the electroencephalograph 200 is an example of a "biological information sensor "of the present disclosure.

As illustrated in Figure 1, the electroencephalogram recording device 100 and the electroencephalograph 200 are configured to perform wireless communication with each other. The electroencephalogram recording device 100 is configured as, for example, a portable terminal having a display screen 101, such as a smartphone. Also, the electroencephalograph 200 is configured as, for example, a wearable terminal worn on a head of a subject X.

In the embodiment, the electroencephalogram recording device 100 is used to record an electroencephalogram as the biological information on the subject X carrying out one or more tasks such as a test or assignment that may be a mental or physical load, and is imposed to check cognitive functions, for example. The electroencephalogram recording device 100 has functions as illustrated in Figure 2 below.

Figure 2 is an exemplary and schematic block diagram illustrating the functions of the electroencephalogram recording device 100 according to the embodiment. As illustrated in Figure 2, the electroencephalogram recording device 100 according to the embodiment includes an input receiver 110, an acquisition processor 120, a recording processor 130, and a display processor 140.

The input receiver 110 receives an input of an operation to the electroencephalogram recording device 100. The input of the operation is performed via a touch panel (not illustrated) that is provided on the display screen 101 by, for example, a user using the electroencephalogram recording device 100 (for example, an observer observing the subject X).

The acquisition processor 120 acquires an electroencephalogram of the subject X which is detected by the electroencephalograph 200. The recording processor 130 records the electroencephalogram acquired by the acquisition processor 120.

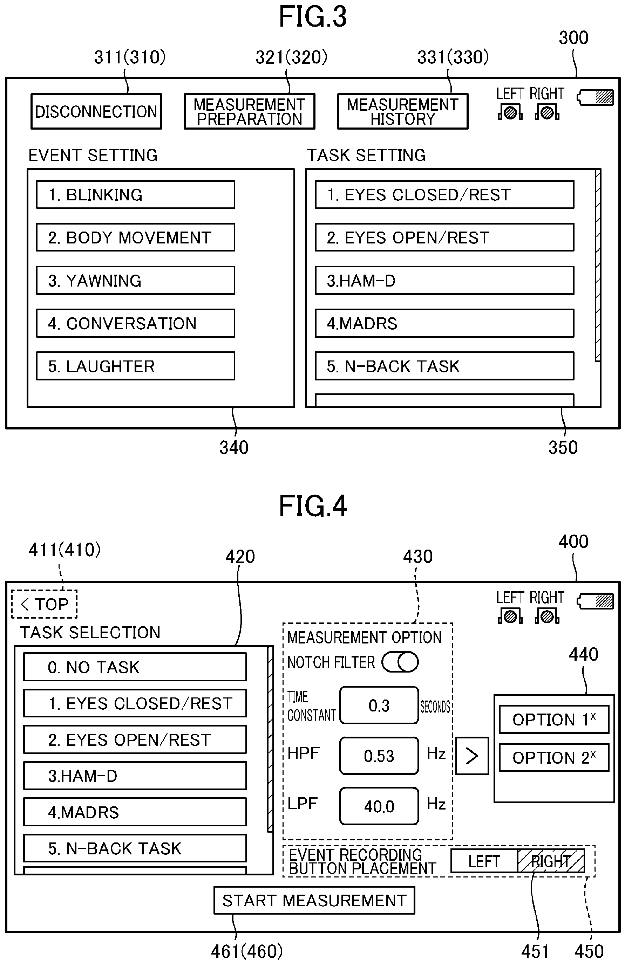

Here, in the embodiment, the display processor 140 is configured to be able to display images as illustrated in Figure 3 to Figure 8 below on the display screen 101.

First, the display processor 140 displays a top image 300 as illustrated in Figure 3 below on the display screen 101 when connection (paring) of the electroencephalogram recording device 100 and the electroencephalograph 200 is established.

Figure 3 is an exemplary and schematic diagram illustrating an example of the top image 300 according to the embodiment.

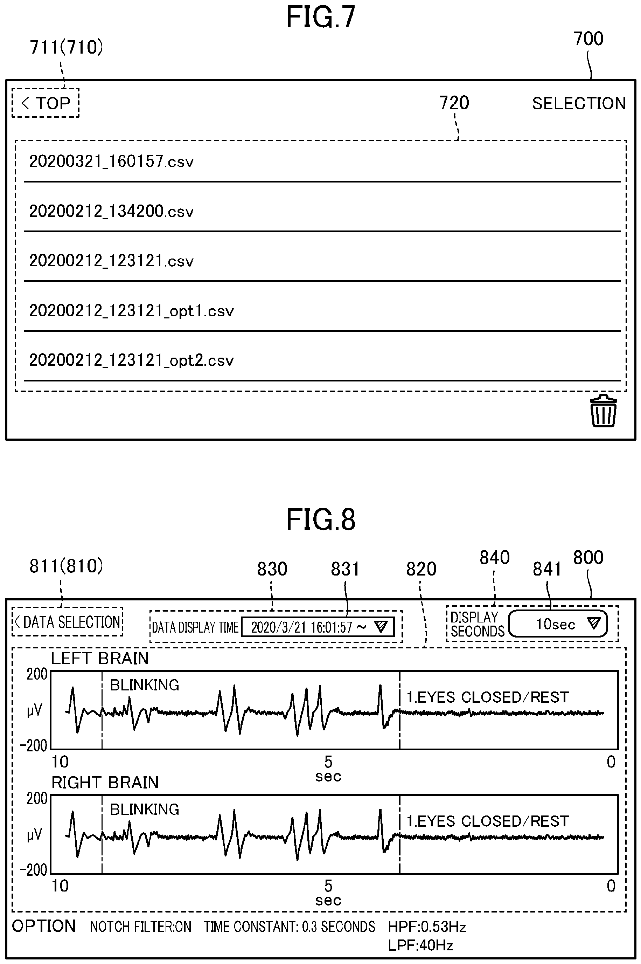

In the region 310, a user interface (button) 311 is displayed, which receives an input of an operation that cancels connection of the electroencephalogram recording device 100 and the electroencephalograph 200. In the region 320, a user interface (button) 321 is displayed, which receives an input of an operation that switches the top image 300 displayed at present to a preparation image 400 (see Figure 4) described later. In the region 330, a user interface (button) 331 is displayed, which receives the input of the operation that switches the top image 300 displayed at present to a history image 700 (see Figure 7) described later.

Further, in the region 340, a user interface is displayed, which receives an input of an operation that sets an event that may occur in the subject X who is carrying out a predetermined task. An event is a particular event that occurs in the subject X while executing a task and may result in noise in the recorded electroencephalogram. In the example illustrated in Figure 3, five events are set, such as blinking, body movement, yawning, conversation, and laughter. The user of the electroencephalogram recording device 100 can additionally set a new event that is different from blinking, body movement, yawning, conversation, and laughter, or change at least one of blinking, body movement, yawning, conversation, and laughter to another event by inputting a predetermined operation via the region 340.

Further, in the region 350, a user interface is displayed, which receives an input of an operation that sets a task to be carried out by the subject X at a time of recording of an electroencephalogram. In the example illustrated in Figure 3, at least five events are set, such as eyes closed/rest, eyes open/rest, HAM-D, MADRS, and N-back tasks. By inputting a predetermined operation via the region 350, the user of the electroencephalogram recording device 100 can additionally set a new task different from the eyes closed/rest, eyes opened/rest, HAM-D, MADRS, and N-back tasks, or change at least one of the eyes closed/rest, eyes opened/rest, HAM-D, MADRS, and N-back tasks to another task.

In the embodiment, when the user interface (button) 321 displayed in the region 320 is operated, the display processor 140 switches to the preparation image 400 as illustrated in Figure 4 as follows from the top image 300 and displays the preparation image 400 on the display screen 101.

Figure 4 is an exemplary and schematic diagram illustrating an example of the preparation image 400 according to the embodiment. As illustrated in Figure 4, the preparation image 400 includes at least six regions 410, 420, 430, 440, 450, and 460.

The region 410 displays a user interface (button) 411 that receives an input of an operation that returns the preparation image 400 displayed at present to the top image 300 (see Figure 3).

Further, in the region 420, user interfaces (buttons) are displayed, which receive an input of an operation that inputs a designation operation that designates a task to be carried out by the subject X at the time of recording an electroencephalogram. The user of the electroencephalogram recording device 100 can select one task from the task that is set in the region 350 (see Figure 3) of the top image 300 and a task of no-task that does not cause the subject to perform any task by performing a designation operation by selection of the user interface of the region 420. The user of the electroencephalogram recording device 100 selects one task via the region 420, and then instructs the subject X to carry out the one task.

Further, in the region 430, a user interface is displayed, which receives an input of an operation that sets a measurement option concerning recording of an electroencephalogram, and in the region 440, a user interface is displayed, which receives an input of an operation that selects a preset of the measurement option. The user of the electroencephalogram recording device 100 can set on/off, a time constant, a frequency of HPF, and a frequency of LPF of a notch filter that is used at the time of recording the electroencephalogram, by inputting a predetermined operation via the regions 430 and 440.

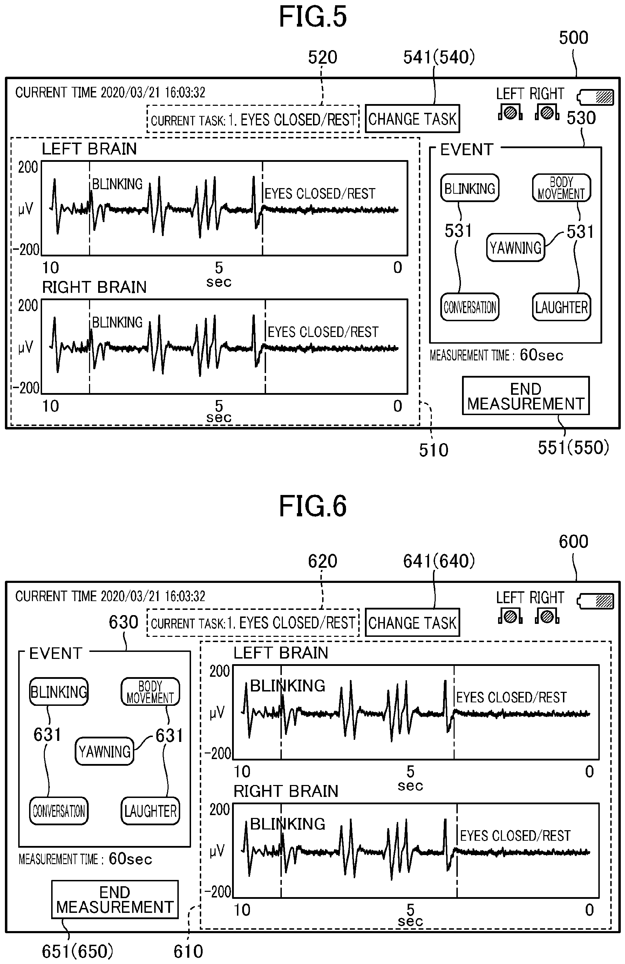

Further, in the region 450, a user interface (button) 451 is displayed, which receives an input of an operation that selects a dominant hand of the user of the electroencephalogram recording device 100. The region 450 is an example of a "fifth region" of the present disclosure, and the user interface 451 is an example of a "third user interface" of the present disclosure. The user of the electroencephalogram recording device 100 can selectively switch between displaying a recording image 500 (see Figure 5) for a right-hander described later on the display screen 101 and displaying a recording image 600 (see Figure 6) for a left-hander described later on the display screen 101 at a time of recording the electroencephalogram, task, and event, by inputting a predetermined operation via the user interface 451 of the region 450.

Further, in the region 460, a user interface (button) 461 for switching the preparation image 400 displayed at present to a recording image 500 (see Figure 5) or a recording image 600 (see Figure 6) described later for recording an electroencephalogram, a task and an event is displayed.

For example, in the embodiment, when the user's dominant hand is selected as the right hand via the user interface 451 of the region 450 and then, an operation to the user interface 461 of the region 460 is input, the display processor 140 switches from the preparation image 400 to the recording image 500 as illustrated in Figure 5 as follows and displays the recording image 500 on the display screen 101. With this, the recording processor 130 starts recording the electroencephalogram.

Figure 5 is an exemplary and schematic diagram illustrating an example of the recording image 500 for a right-hander according to the embodiment. As illustrated in Figure 5, the recording image 500 includes at least five regions 510, 520, 530, 540 and 550.

In the region 510, a change over time of the electroencephalogram of the subject X is displayed in a form of a graph. The region 510 is an example of a "first region" of the present disclosure. In an example illustrated in Figure 5, two kinds of changes over time that are a change over time of an electroencephalogram of a right brain, and a change over time of an electroencephalogram of a left brain are displayed. This means that the electroencephalograph 200 including two electrodes that are an electrode for detecting the electroencephalogram of a right brain, and an electrode for detecting the electroencephalogram of a left brain is used as an example, in the embodiment. The recording processor 130 records an electroencephalogram showing a change over time displayed in the region 510 while the recording image 500 is displayed on the display screen 101.

In the region 520, a name of a present task is displayed. The region 520 is an example of a "second region" of the present disclosure. The name of the task displayed in the region 520 corresponds to a name of a task which is designated by the designation operation via the region 420 of the preparation image 400, and which the user of the electroencephalogram recording device 100 instructs the subject X to carry out. Further, the name of the task displayed in the region 520 is displayed in a manner in which a timing at which the task was started can be identified, on the graph showing the change over time of the electroencephalogram displayed in the region 510. In the embodiment, the recording processor 130 records the task displayed in the region 520 in association with the electroencephalogram while the recording image 500 is displayed on the display screen 101.

Further, in the region 530, user interfaces (buttons) 531 are displayed, which receive an input of a determination operation that determines an event that occurs in the subject X who is carrying out a task. The region 530 is an example of a "third region" of the present disclosure, and the user interfaces 531 are examples of a "first user interface" of the present disclosure. In the example illustrated in Figure 5, five events similar to those in the example illustrated in Figure 1 are displayed in the region 530. The user of the electroencephalogram recording device 100 observes the subject X who is carrying out the task, and each time an event occurs in the subject X, the user can select the user interface 531 corresponding to the event. In this case, each time the user interface 531 is selected, the recording processor 130 records an event corresponding to the selected user interface 531 in association with the electroencephalogram and the task. Note that the name of the event determined by the determination operation is displayed in a manner in which the timing at which the event occurred can be identified, on a graph illustrating a change over time of the electroencephalogram displayed in the region 510.

Further, in the region 540, a user interface (button) 541 is displayed, which receives an input of a change operation (task changing operation) for changing the task so as to re-perform a designation operation of a task to be carried out by the subject X. The region 540 is an example of a "fourth region" of the present disclosure, and the user interface 541 is an example of a "second user interface" of the present disclosure. When a new task is designated by the task changing operation being input via the user interface 541, the recording processor 130 records the new task in association with the electroencephalogram.

Further, in the region 550, a user interface (button) 551 is displayed, which receives an input of a termination operation. When the termination operation is input via the user interface 551, the recording processor 130 ends recording of the electroencephalogram, the task and the event.

Note that in the embodiment, when the dominant hand of the user is selected as a left hand via the user interface 451 of the region 450 in the preparation image 400 (see Figure 4) and then, the operation to the user interface 461 of the region 460 is input, the display processor 140 switches from the preparation image 400 to the recording image 600 as illustrated in Figure 6 as follows and displays the recording image 600 on the display screen 101 instead of the aforementioned recording image 500.

Figure 6 is an exemplary and schematic diagram illustrating an example of the recording image 600 for a left-hander according to the embodiment. As illustrated in Figure 6, the recording image 600 is basically similar to the aforementioned recording image 500 (see Figure 5) except that only an arrangement of some regions is different.

In other words, the recording image 600 includes at least five regions 610, 620, 630, 640, and 650 that are respectively similar to the aforementioned five regions 510, 520, 530, 540, and 550 (see Figure 5). User interfaces (buttons) 631, 641, and 659 that are respectively displayed in the regions 630, 640, and 650 are also similar to the user interfaces 531, 541, and 551 (see Figure 5) respectively displayed in the regions 530, 540, and 550.

Here, in the recording image 600, the region 630 where the user interfaces 631 that receive an input of a determination operation are displayed, and the region 650 where the user interface 651 that receives an input of a termination operation is displayed are located on a left side from the region 610 where a change over time of an electroencephalogram is displayed. This is opposite to a left-right positional relationship of the regions 510, 530, and 650 in the aforementioned recording image 500 (see Figure 5). According to the positional relationship as illustrated in Figure 6, it is easy for a left-handed user to perform a determination operation and a termination operation, and according to the positional relationship as illustrated in Figure 5, it is easy for a right-handed user to perform a determination operation and a termination operation.

Returning to Figure 3, in the embodiment, when the user interface (button) 331 displayed in the region 330 is operated, the display processor 140 switches to a history image 700 as illustrated in Figure 7 as follows from the top image 300 and displays the history image 700 on the display screen 101.

Figure 7 is an exemplary and schematic diagram illustrating an example of the history image 700 according to the embodiment. As illustrated in Figure 7, the history image 700 includes at least two regions 710 and 720.

In the region 710, a user interface (button) 711 is displayed, which receives an input of an operation that returns the history image 700 that is displayed at present to the top image 300 (see Figure 3).

Further, in the region 720, a list of recorded data is displayed. The user of the electroencephalogram recording device 100 inputs a predetermined operation via the region 720 and can select one data from the list of the data displayed in the region 720. In this case, in order to cause the user to confirm a content of the selected data, the display processor 140 switches to a confirmation image 800 as illustrated in Figure 8 as follows from the history image 700 and displays the confirmation image 800 on the display screen 101.

Figure 8 is an exemplary and schematic diagram illustrating an example of the confirmation image 800 according to the embodiment. As illustrated in Figure 8, the confirmation image 800 includes at least four regions 810, 820, 830, and 840.

In the region 810, a user interface (button) 811 is displayed, which receives an input of an operation that returns the confirmation image 800 displayed at present to the history image 700 (see Figure 7). Further, in the region 820, at least a part of the content (for example, correspondence of the electroencephalogram, task and event) of the data selected in the history image 700 is displayed in the form of a graph. In an example illustrated in Figure 8, (at least a part of) the recorded electroencephalogram is displayed in the form of a graph, and the recorded task and event are displayed on the graph in the manner in which the timing at which the task was started and the timing at which the event occurred can be identified.

Further, in the regions 830 and 840, user interfaces 831 and 841 that receive an input of an operation that changes data to be displayed in the region 810 are respectively displayed. For example, the user of the electroencephalogram recording device 100 can select how many seconds of data is displayed in the region 810 by inputting a predetermined operation via the user interface 841 of the region 840, and can cause data that is not within a range of the region 810 at present to be displayed within the range of the region 810 by inputting a predetermined operation via the user interface 831 in the region 830.

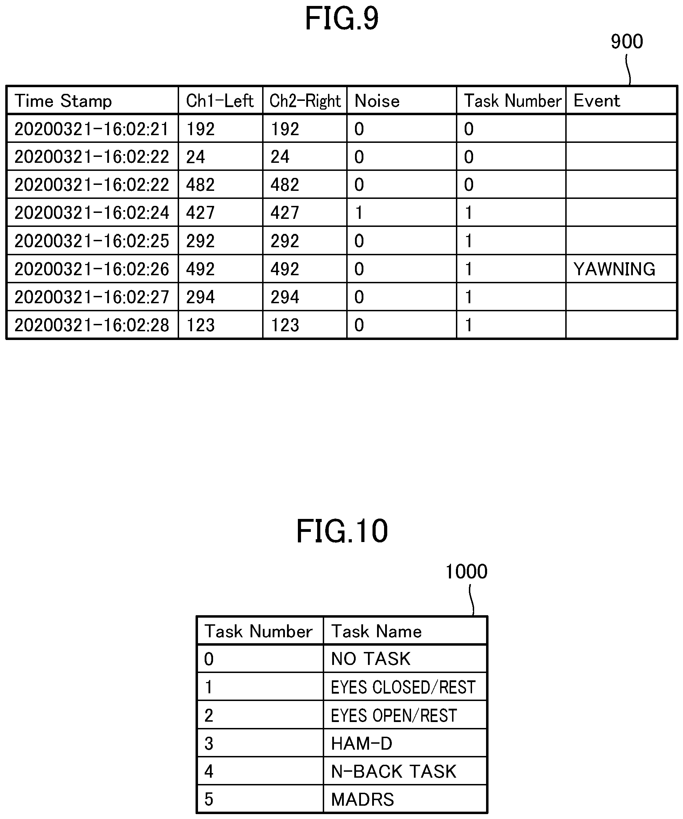

Note that in the embodiment, the recording processor 130 records various data in the format as illustrated in Figure 9 as follows, for example.

Figure 9 is an exemplary and schematic diagram illustrating an example of a recording format of data according to the embodiment.

As illustrated in Figure 9, the recording processor 130 records various data in a format like Table 900. In an example illustrated in Figure 9, data on a total of six items are associated as Table 900, which are a value of the electroencephalogram of the left brain (Ch1-Left), a value of the electroencephalogram of the right brain (Ch2-Right), an identification number of the task (Task Number), a name of the event (Event), presence or absence of noise (Noise), and timing at which the data on these five items were acquired (Time Stamp).

Note that in Table 900 illustrated in Figure 9, the name of a task is not directly recorded, but the identification number of the task is recorded. Therefore, in the embodiment, correspondence of the identification number of the task and the name of the task is recorded in a format as illustrated in Figure 10 as follows.

Figure 10 is an exemplary and schematic diagram illustrating an example of a recording format of a task according to the embodiment.

As illustrated in Figure 10, in the embodiment, the identification number of a task (Task Number) and the name of the task (Task Name) are associated as Table 1000. The display processor 140 displays the aforementioned confirmation image 800 (see Figure 8) on the display screen 101 based on Table 1000 and Table 900 described above (see Figure 9).

In this way, the electroencephalogram recording device 100 according to the embodiment can record the correspondence of the electroencephalogram, task and event, and cause the user to confirm the recorded data, by displaying various images on the display screen 101, and receiving an input of an operation of the user (observer observing the subject X).

Hereinafter, a flow of a process executed by the electroencephalogram recording device 100 according to the embodiment will be described.

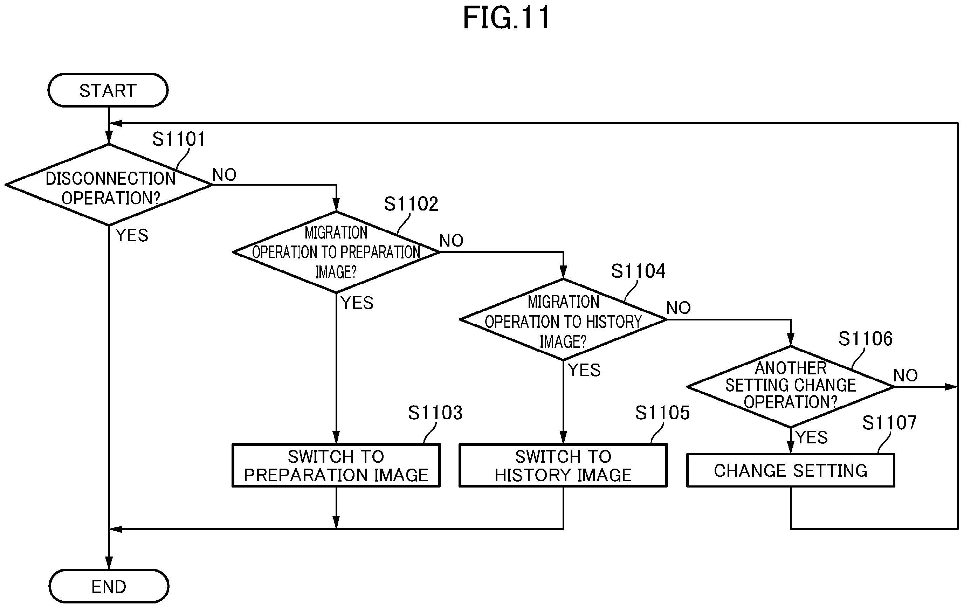

Figure 11 is an exemplary and schematic diagram illustrating the process that is executed when the top image 300 (see Figure 3) is displayed in the embodiment.

In an example illustrated in Figure 11, first, in step S1101, the input receiver 110 determines whether or not a disconnection operation is input. The disconnection operation is an operation that cancels the connection between the electroencephalogram recording device 100 and the electroencephalograph 200, and is input via the user interface 311 of the region 310 in the top image 300 (see Figure 3).

In step S1101, when it is determined that the disconnection operation is input, the process ends. On the other hand, in step S1101, when it is determined that the disconnection operation is not input, the process proceeds to step S1102.

Then, in step S1102, the input receiver 110 determines whether or not a migration operation to the preparation image 400 (see Figure 4) is input. The migration operation to the preparation image 400 is an operation that is input via the user interface 321 of the region 320 in the top image 300 (see Figure 3).

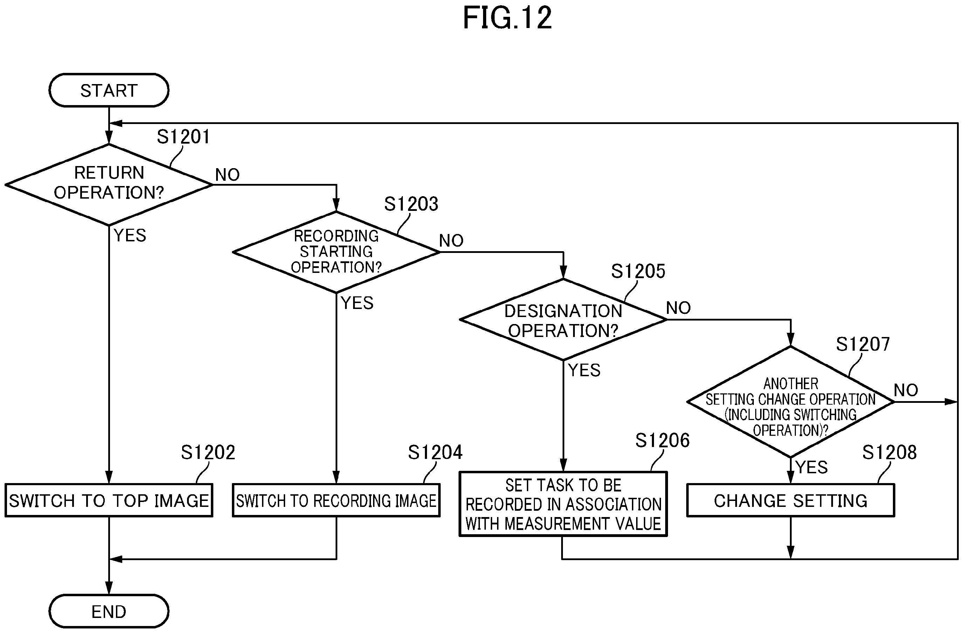

In step S1102, when it is determined that the migration operation to the preparation image 400 (see Figure 4) is input, the process proceeds to step S1103. Then, in step S1103, the display processor 140 switches the image that is displayed on the display screen 101 to the preparation image 400 from the top image 300 (see Figure 3) displayed at present. Then, when switching to the preparation image 400 is completed, the process illustrated in Figure 11 ends, and the process migrates to a process illustrated in Figure 12 (details are described later).

On the other hand, in step S1102, when it is determined that the migration operation to the preparation image 400 (see Figure 4) is not input, the process proceeds to step S1104. Then, in step S1104, the input receiver 110 determines whether or not a migration operation to the history image 700 (see Figure 7) is input. The migration operation to the history image 700 is an operation that is input via the user interface 331 of the region 330 in the top image 300 (see Figure 3).

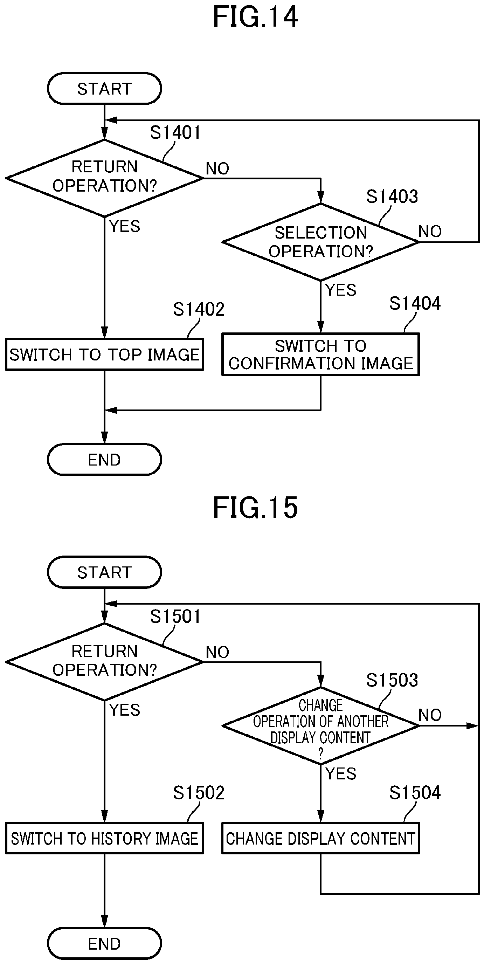

In step S1104, when it is determined that the migration operation to the history image 700 (see Figure 7) is input, the process proceeds to step S1105. Then, in step S1105, the display processor 140 switches the image that is displayed on the display screen 101 to the history image 700 from the top image 300 (see Figure 3) displayed at present. Then, when the switching to the history image 700 is completed, the process illustrated in Figure 11 ends, and the process migrates to a process illustrated in Figure 14 (details are described later).

On the other hand, in step S1104, when it is determined that the migration operation to the history image 700 (see Figure 7) is not input, the process proceeds to step S1106. Then, in step S1106, the input receiver 110 determines whether or not another setting change operation is input. The other setting change operation is an operation that is input via the regions 340 and 350 or the like in the top image 300 (see Figure 3).

In step S1106, when it is determined that the setting change operation is not input, the process returns to step S1101. On the other hand, in step S1106, when it is determined that the setting change operation is input, the process proceeds to step S1107. Then, in step S1107, the display processor 140 changes the setting in response to the input setting change operation and changes a display content of the top image 300 (see Figure 3) as necessary. Then, the process returns to step S1101.

Figure 12 is an exemplary and schematic diagram illustrating a process that is executed when the preparation image 400 (see Figure 4) is displayed in the embodiment.

In an example illustrated in Figure 12, first, in step S1201, the input receiver 110 determines whether or not a return operation is input. The return operation refers to an operation that is input via the user interface 411 of the region 410 in the preparation image 400 (see Figure 4).

In step S1201, when it is determined that the return operation is input, the process proceeds to step S1202. Then, in step S1202, the display processor 140 switches the image that is displayed on the display screen 101 to the top image 300 (see Figure 3) from the preparation image 400 (see Figure 4) displayed at present. Then, when switching to the top image 300 is completed, the process illustrated in Figure 12 ends, and the process migrates to the process illustrated in Figure 11.

On the other hand, in step S1201, when it is determined that the return operation is not input, the process proceeds to step S1203. Then, in step S1203, the input receiver 110 determines whether or not a recording starting operation is input. The recording starting operation is an operation that is input via the user interface 461 of the region 460 in the preparation image 400 (see Figure 4).

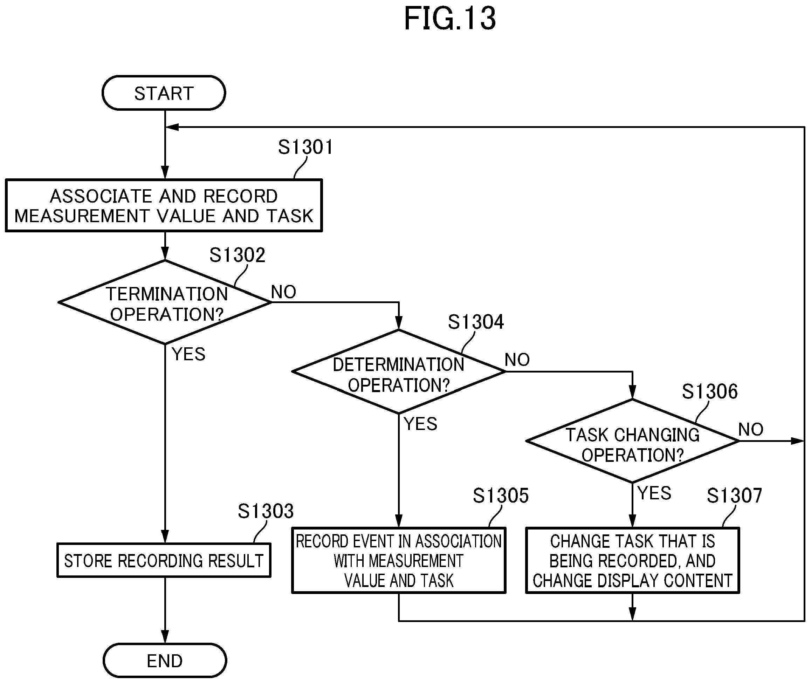

In step S1203, when it is determined that the recording starting operation is input, the process proceeds to step S1204. Then, in step S1204, the display processor 140 switches the image that is displayed on the display screen 101 to the recording image 500 (see Figure 5) or the recording image 600 (see Figure 6) from the preparation image 400 (see Figure 4) that is displayed at present. Then, when the switching to the recording image 500 or 600 is completed, the process illustrated in Figure 12 ends, and the process migrates to a process illustrated in Figure 13 (details are described later).

On the other hand, in step S1203, when it is determined that the recording starting operation is not input, the process proceeds to step S1205. Then, in step S1205, the input receiver 110 determines whether or not a designation operation is input. The designation operation is an operation that is input via the region 420 in the preparation image 400 (see Figure 4).

In step S1205, when it is determined that the designation operation is input, the process proceeds to step S1206. Then, in step S1206, the display processor 140 sets a task to be recorded in association with a measurement value of an electroencephalogram that starts to be recorded in response to a future recording starting operation and changes the display content of the preparation image 400 (see Figure 4) as necessary. Then, the process returns to step S1201.

On the other hand, in step S1205, when it is determined that the designation operation is not input, the process proceeds to step S1207. Then, in step S1207, the input receiver 110 determines whether or not other setting change operations (including a switching operation) are input. The other setting change operations are operations that are input via the regions 430, 440, and 450 in the preparation image 400 (see Figure 4). Here, the operation input via the user interface 451 of the region 450 is expressed as the switching operation in the sense that the operation input via the user interface 451 of the regions 450 is the operation that switches whether the recording image 500 for a right-hander (see Figure 5) is displayed or the recording image 600 for a left-hander (see Figure 6) is displayed on the display screen 101 at the time of recording of the electroencephalogram, task and event.

In step S1207, when it is determined that the setting change operation is not input, the process returns to step S1201. On the other hand, in step S1207, when it is determined that the setting change operation is input, the process proceeds to step S1208. Then, in step S1208, the display processor 140 changes setting in response to the input setting change operation, and changes the display content of the preparation image 400 (see Figure 4) as necessary. Then, the process returns to step S1201.

Fig. 13 is an exemplary and schematic diagram illustrating a process that is executed when the recording image 500 (see Figure 5) or the recording image 600 (see Figure 6) is displayed in the embodiment. As described above, whether the recording image 500 or 600 is displayed is switched in response to the switching operation input in step S1207 in Figure 12, for example.

In an example illustrated in Figure 13, the recording processor 130 associates and records the measurement value of the electroencephalogram acquired by the acquisition processor 120, and the task designated by the designation operation input in step S1205 in Figure 12, for example. At this time, the display processor 140 displays the data that are being recorded in the region 510 of the recording image 500 (see Figure 5) or the region 610 of the recording image 600 (see Figure 6).

Then, in step S1302, the input receiver 110 determines whether or not a termination operation is input. The termination operation is an operation that is input via the user interface 551 of the region 550 in the recording image 500 (see Figure 5) or the user interface 651 of the region 650 in the recording image 600 (see Figure 6).

In step S1302, when it is determined that the termination operation is input, the process proceeds to step S1303. Then, in step S1303, the recording processor 130 stores the recording result of the data up to the present point of time in the format like Table 900 (see Figure 9) mentioned above, for example. Then, the process ends.

On the other hand, in step S1302, when it is determined that the termination operation is not input, the process proceeds to step S1304. Then, in step S1304, the input receiver 110 determines whether or not the determination operation is input. The determination operation is an operation that is input via the user interface 531 of the region 530 in the recording image 500 (see Figure 5), or the user interface 631 of the region 630 in the recording image 600 (see Figure 6).

When it is determined that the determination operation is input, in step S1304, the process proceeds to step S1305. Then, in step S1305, the recording processor 130 records the event determined by the determination operation in association with the measurement value of the electroencephalogram and the task that are being recorded. At this time, the display processor 140 displays the recorded event in the region 510 of the recording image 500 (see Figure 5), or in the region 610 of the recording image 600 (see Figure 6). Then, the process returns to step S1301.

On the other hand, in step S1305, when it is determined that the determination operation is not input, the process proceeds to step S1306. Then, in step S1306, the input receiver 110 determines whether or not a task change operation is input. The task change operation is an operation that is input via the user interface 541 of the region 540 in the recording image 500 (see Figure 5), or the user interface 641 of the region 640 in the recording image 600 (see Figure 6).

When it is determined that the task change operation is not input, in step S1306, the process returns to step S1301. On the other hand, when it is determined that the task change operation is input, in step S1306, the process proceeds to step S1307. Then, in step S1307, the recording processor 130 changes the task that is being recorded and changes the display content in the region 520 of the recording image 500 (see Figure 5), or in the region 620 of the recording image 600 (see Figure 6). At this time, the display processor 140 also changes the task that is displayed in the region 510 of the recording image 500, or in the region 610 of the recording image 600. Then, the process returns to step S1301.

Figure 14 is an exemplary and schematic diagram illustrating a process that is executed when the history image 700 (see Figure 7) is displayed in the embodiment.

In an example illustrated in Figure 14, first, in step S1401, the input receiver 110 determines whether or not the return operation is input. The return operation is an operation that is input via the user interface 711 of the region 710 in the history image 700 (see Figure 7).

In step S1401, when it is determined that the return operation is input, the process proceeds to step S1402. Then, in step S1402, the display processor 140 switches the image that is displayed on the display screen 101 to the top image (see Figure 3) from the history image 700 (see Figure 7) displayed at present. Then, when the switching to the top image 300 is completed, the process illustrated in Figure 14 ends, and the process migrates to the process illustrated in Figure 11.

On the other hand, in step S1401, when it is determined that the return operation is not input, the process proceeds to step S1403. Then, in step S1403, the input receiver 110 determines whether or not a selection operation is input. The selection operation is an operation that is input via the region 720 in the history image 700 (see Figure 7).

When it is determined that the selection operation is not input, in step S1403, the process returns to step S1401. On the other hand, in step S1403, when it is determined that the selection operation is input, the process proceeds to step S1404. Then, in step S1404, the display processor 140 switches the image that is displayed on the display screen 101 to the confirmation image 800 (see Figure 8) from the history image 700 (see Figure 7) displayed at present. Then, when the switching to the confirmation image 800 is completed, the process illustrated in Figure 14 ends, and the process migrates to a process illustrated in Figure 15 as follows.

Figure 15 is an exemplary and schematic diagram illustrating a process that is executed when the confirmation image 800 (see Figure 8) is displayed in the embodiment.

In an example illustrated in Figure 15, first, in step S1501, the input receiver 110 determines whether or not a return operation is input. The return operation is an operation that is input via the user interface 811 of the region 810 in the confirmation image 800 (see Figure 8).

In step S1501, when it is determined that the return operation is input, the process proceeds to step S1502. Then, in step 1502, the display processor 140 switches the image that is displayed on the display screen 101 to the history image 700 (see Figure 7) from the confirmation image 800 (see Figure 8) displayed at present. Then, when the switching to the history image 700 is completed, the process illustrated in Figure 15 ends, and the process migrates to the process illustrated in Figure 14.

On the other hand, when it is determined that the return operation is not input, in step S1501, the process proceeds to step S1503. Then, in step S1503, the input receiver 110 determines whether or not a change operation of other display content is input. The change operation of the other display content is an operation that is input via the user interface 831 of the region 830 and the user interface 841 of the region 840 in the confirmation image 800 (see Figure 8).

When it is determined that the change operation of the display content is not input, in step S1503, the process returns to step S1501. On the other hand, when it is determined that the change operation of the display content is input, in step S1503, the process proceeds to step S1504. Then, in step S1504, the display processor 140 changes the display content in the region 820 of the confirmation image 800 (see Figure 8), in response to the change operation of the display content that is input. Then, the process ends.

As described above, the electroencephalogram recording device 100 according to the embodiment includes the acquisition processor 120 and the recording processor 130. The acquisition processor 120 acquires the electroencephalogram of the subject X, which is detected by the electroencephalograph 200. The recording processor 130 records the electroencephalogram acquired by the acquisition processor 120, and further records a task in association with the electroencephalogram, when the designation operation that designates the task to be carried out by the subject X is performed by the observer who observes the subject X during recording of the electroencephalogram.

According to the above-described configuration, the electroencephalogram and tasks can be easily associated and recorded by simply performing the designation operation in real time during recording of the electroencephalogram, unlike the case of retrospectively recording which task was carried out at what timing, after completion of recording of the electroencephalogram, for example.

Further, in the embodiment, when the observer performs a determination operation that determines an event that occurs in the subject X during recording of an electroencephalogram and a task, the recording processor 130 further records the event in association with the electroencephalogram and the task.

According to the above-described configuration, an event can also be easily associated and recorded, in addition to an electroencephalogram and a task by simply performing the determination operation in real time during recording of the electroencephalogram and the task, unlike the case of retrospectively recording which event was carried out at what timing after completion of recording of the electroencephalogram and the task, for example. In other words, one observer can simultaneously record an event (for example, blinking or the like) occurring to the subject, in real time while causing the subject to carry out a task such as a cognitive test. Accordingly, in recording of an electroencephalogram, noise, artifact or the like can be easily identified. Further, in this case, the observer can work while watching both the electroencephalogram and an action and a motion of the subject, and therefore, working efficiency can be enhanced.

Note that in the embodiment, the electroencephalogram recording device 100 incudes the display processor 140. The display processor 140 displays the recording image 500 (or 600) that includes the region 510 (or 610) where a change over time of an electroencephalogram is displayed, the region 520 (or 620) where the name of a task is displayed, and the region 530 (or 530) where the user interface 531 (or 631) that receives an input of the determination operation is displayed, on the display screen 101.

According to the above-described configuration, determination of an event to be recorded in association with an electroencephalogram and a task can be simply performed visually.

Note that in the embodiment, the recording image 500 (or 600) further includes the region 540 (or 640) where the user interface 541 (or 641) is displayed, which receives an input of a change operation that changes the task so as to re-perform a designation operation.

According to the above-described configuration, a change of the task to be recorded in association with an electroencephalogram can be simply performed visually.

Further, in the embodiment, before displaying the recording image 500 (or 600) on the display screen 101, the display processor 140 displays the preparation image 400 including the region 450 where the user interface 451 is displayed, which receives an input of a switching operation that switches whether the region 530 (or 630) is disposed on a left side or a right side of the region 510 (or 620) in the recording image 500 (or 600), on the display screen 101.

According to the above-described configuration, by the switching operation using the preparation image 400, it is possible to easily select the recording image 500 where a right-handed user easily performs a determination operation, and the recording image 600 where a left-handed user easily performs a determination operation.

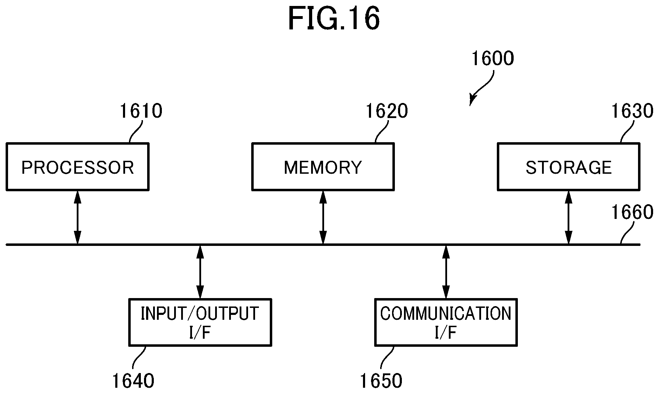

In the end, a hardware configuration of the electroencephalogram recording device 100 according to the aforementioned embodiment is described. The electroencephalogram recording device 100 according to the embodiment is realized by a computer 1600 having a hardware configuration as shown in Figure 16 as follows, for example.

Figure 16 is an exemplary and schematic block diagram illustrating the hardware configuration of the computer 1600 that realizes the electroencephalogram recording device 100 according to the embodiment.

As illustrated in Figure 16, the computer 1600 includes a processor 1610, a memory 1620, a storage 1630, an input/output interface (I/F) 1640, and a communication interface (I/F) 1650. These pieces of hardware are connected to a bus 1660.

The processor 1610 is configured as a CPU (Central Processing Unit), for example, and controls operations of respective units of the computer 1600 as a whole.

The memory 1620 includes, for example, a read only memory (ROM) and a random access memory (RAM), and realizes volatile or nonvolatile storage of various data, such as programs executed by the processor 1610, provision of a work region for the processor 1610 to execute the programs, and the like.

The storage 1630 includes, for example, a hard disk drive (HDD) or solid state drive (SSD) and stores various data in a non-volatile manner.

The input/output interface 1640 controls inputs of data to the computer 1600, for example, from input devices (not illustrated) such as a keyboard and a mouse, and outputs of data, for example, to output devices (not illustrated) such as a display and a speaker from the computer 1600.

The communication interface 1650 enables the computer 1600 to execute wired or wireless communication with other devices.

Functions (see Figure 2) of the electroencephalogram recording device 100 according to the embodiment are realized as a function module group by cooperation of the hardware and the software as a result of the processor 1610 executing an electroencephalogram recording program that is pre-stored in the memory 1620 or the storage 1630. However, in the embodiment, a part or a whole of the function module group illustrated in Figure 2 may be realized by only hardware such as a circuit (circuitry) designed exclusively.

Note that the aforementioned electroencephalogram recording program does not necessarily have to be pre-stored in the memory 1620 or the storage 1630. For example, the aforementioned electroencephalogram recording program may be provided as a computer program product recorded in a format installable or a format executable in a computer-readable media such as various magnetic disks such as a flexible disk (FD), or various optical disks such as a digital versatile disk (DVD).

Further, the aforementioned electroencephalogram recording program may be provided or distributed via a network such as the Internet. In other words, the aforementioned recording program may be provided in a form in which it is in a state stored on a computer connected to a network such as the Internet to accept download via the network.

### <Modified Example>

In the aforementioned embodiment, the configuration in which the biological information to be recorded is an electroencephalogram is illustrated. However, the technology of the present disclosure is also applicable to a configuration for recording biological information other than electroencephalograms, such as heartbeats.

Further, in the aforementioned embodiment, the electroencephalogram recording device 100 configured as a portable terminal like a smartphone is illustrated as an example of the biological information recording device. However, in the technology of the present disclosure, the electroencephalogram recording device 100 may be configured as a non-portable terminal.

Further, in the aforementioned embodiment, the case where the designation operation that designates the task to be carried out by the subject is performed by the observer who observes the subject during recording of the biological information is shown, but the entity that performs the designation operation is not limited to the observer, and the designation operation may be performed by a mechanical device. The mechanical device includes, for example, a camera that photographs an entire image of a subject, and a computer including at least one processor. The processor performs the designation operation by arbitrarily designating a task to be carried out by the subject, displays the designated task in an input/output interface (I/F) of the computer, and transfers information on the task to a recording processor to record the information on the task. Thereby, the information on the task can be accurately recorded.

Also, in the aforementioned embodiment, the case where the determination operation that determines an event occurring in the subject is performed by the observer during recording of the biological information is shown, but the determination operation may also be performed by a mechanical device instead of the observer, similarly to the designation operation described above. The mechanical device includes, for example, a camera that photographs an entire image of the subject, and a computer including at least one processor. The processor performs the determination operation by determining an event that occurs in the subject based on a video from the camera, transfers the determination operation to the recording processor, and records the determination operation, for example. Thereby, the determination operation of the event can be accurately recorded.

Although the several embodiments and modified examples of the present disclosure are described thus far, these embodiments and modified examples are presented as examples, and are not intended to limit the scope of the invention. These novel embodiments and modified examples can be carried out in other various modes, and various omissions, replacements, and changes can be performed in the range without departing from the gist of the invention. These embodiments and modified examples are included in the scope and gist of the invention, and are included in the scope of the invention described in the claims and equivalents thereof.

### Reference Signs List

- 100: biological information recording device
- 101: display screen
- 120: acquisition processor
- 130: recording processor
- 140: display processor
- 200: electroencephalograph
- 400: preparation image
- 450: region (fifth region)
- 451: user interface (second user interface)
- 500, 600: recording image
- 510, 610: region (first region)
- 520, 620: region (second region)
- 530, 630: region (third region)
- 531, 631: user interface (first user interface)
- 540, 640: region (fourth region)
- 541 641: user interface (second user interface)

## Claims

1. A biological information recording device, comprising:
an acquisition processor configured to acquire biological information on a subject, which is detected by a biological information sensor; and
a recording processor configured to record the biological information acquired by the acquisition processor, and to further record, when a designation operation that designates a task to be carried out by the subject is performed by an observer who observes the subject during recording of the biological information, the task in association with the biological information.

2. The biological information recording device according to claim 1,
wherein when a determination operation that determines an event that occurs in the subject is performed by the observer during recording of the biological information and the task, the recording processor further records the event in association with the biological information and the task.

3. The biological information recording device according to claim 2, further comprising
a display processor configured to display a recording image on a display screen, the recording image including a first region where a change over time of the biological information is displayed, a second region where a name of the task is displayed, and a third region where a first user interface that receives an input of the determination operation is displayed, during recording of the biological information and the task.

4. The biological information recording device according to claim 3,
wherein the recording image further includes a fourth region where a second user interface that receives an input of a change operation that changes the task to re-perform the designation operation is displayed.

5. The biological information recording device according to claim 3 or 4,
wherein the display processor displays a preparation image on the display screen before displaying the recording image on the display screen, the preparation image including a fifth region where a third user interface, which receives an input of a switching operation that switches whether the third region is disposed on a left side or a right side of the first region in the recording image, is displayed.

6. The biological information recording device according to any one of claims 1 to 5,
wherein the biological information includes an electroencephalogram detected by an electroencephalograph as the biological information sensor.

7. A biological information recording program for causing a computer to execute:
acquiring biological information on a subject, which is detected by a biological information sensor; and
recording the acquired biological information, and further recording, when a designation operation that designates a task to be carried out by the subject is performed by an observer who observes the subject during recording of the biological information, the task in association with the biological information.

8. The biological information recording program according to claim 7, for causing the computer to execute,
when a determination operation that determines an event that occurs in the subject is performed by the observer during recording of the biological information and the task, further recording the event in association with the biological information and the task.

9. The biological information recording program according to claim 8, for causing the computer to execute
displaying a recording image on a display screen, the recording image including a first region where a change over time of the biological information is displayed, a second region where a name of the task is displayed, and a third region where a first user interface that receives an input of the determination operation is displayed, during recording of the biological information and the task.

10. The biological information recording program according to claim 9,
wherein the recording image further includes a fourth region where a second user interface that receives an input of a change operation that changes the task to re-perform the designation operation is displayed.

11. The biological information recording program according to claim 9 or 10, for causing the computer to execute
displaying a preparation image on the display screen before displaying the recording image on the display screen, the preparation image including a fifth region where a third user interface, which receives an input of a switching operation that switches whether the third region is disposed on a left side or a right side of the first region in the recording image, is displayed.

12. The biological information recording program according to any one of claims 7 to 11,
wherein the biological information includes an electroencephalogram detected by an electroencephalograph as the biological information sensor.
